# EUROPÄISCHE PATENTSCHRIFT

(11) **EP 2 648 122 B1**
(45) Veröffentlichungstag und Bekanntmachung des Hinweises auf die Patenterteilung: **28.06.2017**
(21) Anmeldenummer: 12163393.7
(22) Anmeldetag: 05.04.2012
(51) Int. Cl.: G06F 19/00

(54) **Verfahren zum Laden von medizinischen Bilddaten sowie Vorrichtung zur Durchführung des Verfahrens**
Method for loading medical image data and device for executing the method
Procédé de chargement de données d'images médicales ainsi que le dispositif de réalisation du procédé

(43) Veröffentlichungstag der Anmeldung: 09.10.2013
(73) Patentinhaber: Siemens Healthcare GmbH, 91052 Erlangen (DE)
(72) Erfinder: König, Helmut, 91054 Erlangen (DE); Dorn, Karlheinz, 90562 Kalchreuth (DE); Ukis, Vladyslav, 90489 Nürnberg (DE)

(56) Entgegenhaltungen:
- WO-A1-99/16250
- US-A1- 2009 003 270
- US-A1- 2009 132 636

## Beschreibung

Die Erfindung betrifft ein Verfahren zum Laden von medizinischen Bilddaten, um sie einem Bildbearbeitungsprogramm zur Verfügung zu stellen, wobei die Bilddaten aus Rohdaten eines medizinischen bildgebenden Systems bei der Aufnahme eines Objekts erzeugt sind und die einem jeweiligen Objekt zugeordneten Bilddaten jeweils Metadaten sowie Pixel-Daten umfassen. Die Erfindung betrifft weiterhin eine Vorrichtung zur Durchführung eines solchen Verfahrens.

Medizinische Bilddaten können aus den unterschiedlichsten bilderzeugenden Geräten, den sogenannten Modalitäten stammen. Derartige Modalitäten sind beispielsweise Computertomographen, Magnetresonanztomographen, einfache Röntgengeräte zur Erzeugung von Projektionsbildern etc.. Zu einem Patienten können Bilddaten aus verschiedenen Modalitäten vorliegen. Die Bilddaten werden innerhalb einer beispielsweise klinischen Umgebung in einem gemeinsamen Bildverarbeitungssystem (PACS) gespeichert. Zur Bearbeitung werden die Bilddaten aus diesem Bildspeichersystem auf Anforderung von einem jeweiligen lokalen Client-Rechner zur Bildbearbeitung und Bildauswertung angezeigt oder geladen. Das entsprechende Bildbearbeitungsprogramm (Applikation) ist üblicherweise auf einem leistungsstarken Server installiert. Für die Bildbearbeitung stehen hierbei unterschiedliche Applikationen zur Verfügung, die in Abhängigkeit der Anforderungen entsprechend ausgewählt werden.

Die Bilddaten sind üblicherweise nach dem sogenannten DICOM-Standard (DICOM = Digital imaging and communications in medicine) abgespeichert. Die Bilddaten umfassen META-Daten, die konzeptionell den sogenannten "header" darstellen, sowie die eigentlichen die Bildinformationen enthaltenden BildDaten als Pixel-Daten.

Durch die zunehmend höhere Auflösung moderner digitaler bilderzeugender Geräte sind die Bilddaten eines untersuchten Objekts - je nach Modalität - mitunter sehr groß mit einem Speicherbedarf von mehreren Gigabyte.

Diese großen Datenmengen führen zu Beeinträchtigungen bei dem Laden der Daten, beispielsweise in einen Arbeitsspeicher des Servers, auf dem das Bildbearbeitungsprogramm zur Auswertung und Analyse der Bilddaten zugreift. Dies führt teilweise zu Akzeptanzproblemen seitens des medizinischen Personals als Nutzer derartiger Anwendungen.

Aus der US 2009/0132636 A1 ist ein Verfahren zu entnehmen, bei dem sogenannte Ladepläne für eine Lieferkette der Daten an verschiedene Stellen vorgesehen ist, wobei beispielsweise bei Mehrfachanfragen bei unterschiedlichen Client-Rechnern die Daten unter Berücksichtigung von Datenengpässen über geeignete Datenknotenpunkte geleitet werden.

Der Erfindung liegt die Aufgabe zugrunde, ein Verfahren sowie eine Vorrichtung anzugeben, die ein zügigeres und verbessertes Laden der Bilddaten aus einem zentralen Bilddaten-Speicher in einen Arbeitsspeicher ermöglichen.

Die Aufgabe wird gemäß der Erfindung gelöst durch ein Verfahren mit den Merkmalen des Anspruchs 1 sowie durch eine Vorrichtung zur Durchführung des Verfahrens mit den Merkmalen des Anspruchs 10.

Bei dem Verfahren zum Laden von medizinischen Bilddaten werden die Bilddaten einem Datentyp zugeordnet und anschließend wird aus mehreren datentyp-spezifischen Ladestrategien eine dem ermittelten Datentyp zugewiesene Ladestrategie ausgewählt und für das Laden der Bilddaten zur Bereitstellung für das Bildbearbeitungsprogramm (Applikation) herangezogen. Die Bilddaten sind dabei allgemein aus Rohdaten eines medizinischen bildgebenden Systems bei der Aufnahme eines Objekts erzeugt. Das Objekt ist insbesondere eine bestimmte Körperregion eines Patienten, ein bestimmtes Organ oder auch eine Gewebeprobe bei pathologischen Befunden. Die Bilddaten sind dabei nach dem DICOM-Standard in einem zentralen Datenspeicher insbesondere innerhalb eines PACS-Systems abgelegt. Entsprechend weisen die Bilddaten auch Metadaten sowie Pixel-Daten auf, die die eigentlichen Bildinformationen enthalten.

Unter verschiedene "Datentypen" der Bilddaten wird eine unterschiedliche Datenstruktur der Bilddaten verstanden. Je nach Modalität und Anforderung werden die Rohdaten üblicherweise in unterschiedlichen Datenstrukturen gemäß dem DICOM-Standard abgelegt.

Mit dem Verfahren wird nunmehr in einem ersten Schritt analysiert, um welchen Datentyp, also um welche Datenstruktur es sich bei den Bilddaten handelt. Anschließend wird eine auf diesen jeweiligen Datentyp zugeschnittene Ladestrategie ausgewählt, mit der dann die Bilddaten in einen Arbeitsspeicher zur Bereitstellung für die Bildbearbeitung geladen werden. Durch diese datentyp-spezifische Ladestrategie ist ein performance-optimiertes Laden ermöglicht.

Das Verfahren wird dabei mit Hilfe eines Lademoduls, das vorzugsweise auf einem Server, insbesondere einem Multi-Modalitäten-Server hinterlegt ist, durchgeführt. Unter Multi-Modalitäten-Server wird ein Server verstanden, der Bilddateien aus unterschiedlichen Modalitäten verarbeitet. Grundsätzlich können die Bilddaten auf diesem Server abgespeichert sein oder auch an einem entfernten Speicherort. Mit Hilfe des Lademoduls, bei dem es sich um eine Softwarekomponente handelt, wird automatisch zunächst der Datentyp ermittelt und anschließend mit Hilfe eines Datentyp-Zuordnungsmoduls ein Datentyp den Bilddaten des jeweiligen Objekts zugeordnet. Schließlich enthält das Lademodul weiterhin ein Ablauf- oder Workflowmodul, über das der Ladevorgang der Bilddaten gesteuert wird. Insbesondere wählt dieses Ablaufmodul aus vorgegebenen Default-Ladestrategien die für den jeweiligen Datentyp vorgesehene Default-Ladestrategie aus.

Die Datentypen und Datenstrukturen der Bilddaten sind folgenden Gruppen zugeordnet:
a) Volumen-Bilddaten basierend auf einzelnen Schichtaufnahmen, wie sie beispielsweise bei einer Computertomograph-Untersuchung oder auch bei einer Magnetresonanzuntersuchung anfallen;
b) Volumen-Bilddaten basierend auf Multiframe-Bilddaten; Hierbei handelt es sich um Daten, die nach einem speziellen DICOM-Standard gespeichert sind. Bei diesem werden beispielsweise die Bilddaten mehrerer Schichtbilder in einem sogenannten "Vielfach-Rahmen" (Multiframe) zusammengefasst, die gemeinsame Attribute aufweisen, welche in einem gemeinsamen Header zusammengefasst sind.
c) Bilddaten entsprechend dem WSI-Standard innerhalb des DICOM-Standards (WSI = Whole Slide Imaging). Dieser Standard ist insbesondere für Bilder entwickelt, die bei der digitalen Mikroskopie, beispielsweise für pathologische Anwendungen entwickelt wurde. Bei diesen WSI-Bilddaten werden die Daten üblicherweise in sogenannten Kachelpyramiden (Tile-Pyramid) insbesondere mit für die höchste Bildauflösung optionalen Z-Stapel (z-stocks) gespeichert. Bei diesen Kachelpyramiden werden die Bilddaten mit unterschiedlichen Auflösungen in unterschiedlichen "Ebenen" der Pyramide abgespeichert. Die Basis der Pyramide definiert hierbei Bilddaten mit der höchsten Auflösung. Ergänzend ist jede Ebene der Pyramide in einzelne Felder, sogenannte Kacheln unterteilt.
d) Projektions-Bilddaten, wie sie insbesondere bei einer herkömmlichen Röntgenuntersuchung wie beispielsweise der Radiographie erhalten werden.

Die Bilddaten entsprechend dieser Datentypen unterscheiden sich im Hinblick auf ihre Struktur als auch auf ihr Datenvolumen grundlegend, so dass für jeden dieser Typen eine unterschiedliche Ladestrategie, also ein unterschiedlicher Algorithmus zum Laden der Bilder aus dem Bilddatenspeicher zu einer Optimierung der Ladezeit führt. So ist beispielsweise bei den WSI-Daten vorgesehen, dass nicht alle Daten gleichzeitig geladen werden, sondern dass Daten aus einer vordefinierten Ebene mit vordefinierter Auflösung geladen werden. Bei den Volumenbilddaten gemäß den Datentypen a) und b) werden beispielsweise gemäß der Default-Ladestrategie Bilddaten aus der die Bildmitte repräsentierenden Mitte des Bilddatensatzes geladen, da hier die interessierenden Bereiche zu vermuten sind. Bei den einfachen Projektionsbildern gemäß Datentyp d) kann die Default-Ladestrategie schließlich darin bestehen, gleich den gesamten Datensatz vollständig zu laden, da die Bilddatenmengen hier im Vergleich zu den anderen Datentypen sehr gering sind.

In bevorzugter Ausgestaltung wird der Datentyp dabei anhand der in den Bilddaten enthaltenen Meta-Daten bestimmt. Das Datentyp-Zuordnungsmodul analysiert daher die Metadaten, um die Bilddaten einem speziellen Datentyp zuzuweisen. Gemäß dem DICOM-Standard sind nämlich in den Metadaten Informationen enthalten, die Rückschlüsse auf die Modalität und damit den jeweiligen Datentyp zulassen. Insbesondere erfolgt die Kategorisierung und Zuordnung des Datentyps unter Verwendung des DICOM-Attributs "SOP Class UID" (SOP = Service-Object-Pair; UID = United Identifier). Hierdurch werden daher in effektiver Weise bereits vorhandene Informationen in den eigentlichen Bilddaten ausgewertet.

Den Bilddaten sind neben den Metadaten zusätzliche Kontextdaten zugewiesen, wobei ergänzend anhand der Kontextdaten die Default-Ladestrategie spezifiziert und eine angepasste Ladestrategie bestimmt wird. Die zuvor über den Datentyp ermittelte Default-Ladestrategie wird daher anhand der Kontextdaten näher spezifiziert. Bei diesen Kontextdaten handelt es sich um ergänzende Zusatzinformationen, die nicht in den Metadaten enthalten sind. Durch die Auswertung dieser zusätzlichen Kontextinformationen werden daher zusätzliche Informationen berücksichtigt, um ein optimales Ladeergebnis zu erzielen.

Um dies zu ermöglichen ist weiterhin die Erstellung eines gemeinsamen Index zu einer Vielzahl von medizinischen Objekten vorgesehen. Vorzugsweise umfasst der Index alle, zumindest einen Großteil der in der Datenbank hinterlegten Bilddaten(-sätze) zu den jeweiligen Objekten. Den Bilddaten eines jeweiligen Objekts werden über den Index die diesem Objekt zugehörigen Kontextdaten zugeordnet. Auf diesen insbesondere von dem Lademodul erstellten gemeinsamen Index greift das Lademodul bei einer Anforderung, also bei einem Ladevorgang vorzugsweise zu, um hieraus die angepasste Ladestrategie zu ermitteln.

Der Index wird hierbei zweckdienlicherweise in einem eigenen Indexdatenspeicher gespeichert. Die Erstellung des Index für die Bilddaten eines jeweiligen Objektes erfolgt zweckdienlicherweise sobald die Bilddaten von einem bildgebenden medizinischen Gerät an den Server, auf dem das Lademodul installiert ist, übermittelt werden. Die Indexerstellung erfolgt also in der Regel unabhängig von einem speziellen Ladevorgang der Bilddaten zur Bereitstellung für ein Bildbearbeitungsprogramm. Hierzu ist innerhalb des Lademoduls ein Indexersteller zur Erstellung des Index vorgesehen. In den Index werden dabei beispielsweise nach Art einer Tabellenstruktur alle für die Ermittlung einer performance-optimierten Ladestrategie relevanten Informationen zugeordnet zu dem jeweiligen Objekt hinterlegt. Im Index sind neben der Identifizierung beispielsweise des Speicherortes der Bilddaten auch relevante Informationen aus den Metadaten, wie beispielsweise Information über die Modalität, Datum der Aufnahme, zu untersuchendes Organ etc. hinterlegt. Daneben werden unterschiedliche Arten von Kontextdaten in den Index eingeschrieben. Sofern diese bei der erstmaligen Erfassung der Bilddaten noch (nicht) vorliegen, so bleiben die entsprechenden Felder im Index zunächst leer, werden dann jedoch automatisch gefüllt, sobald die entsprechenden Informationen vorliegen.

Als eine erste Gruppe von Kontextinformationen werden Informationen aus einer bereits erfolgten, vorhergehenden Analyse und Auswertung der Bilddaten hinterlegt. Hierzu umfasst das Lademodul zweckdienlicherweise ein Analysemodul, das zur automatischen Extraktion von Kontextdaten aus den Ergebnissen dieser vorhergehenden Auswertung ausgebildet ist.

Diese Gruppe an Kontextdaten umfasst insbesondere Informationen über interessierende Bildbereiche, die sogenannte "Region Of Interest" (ROI). Der interessierende Bereich wird beispielsweise bei einer vorhergehenden Auswertung im Rahmen eines insbesondere automatischen Segmentationsverfahrens identifiziert. Die angepasste Ladestrategie sieht entsprechend vor, derartige zuvor identifizierende Bildbereiche vorzuladen, bevor die weiteren Daten des Bilddatensatzes eingelesen werden. Durch diese Maßnahme wird daher dem medizinischen Personal sofort der relevante Bildbereich zur Ansicht gebracht, so dass eine sofortige Bearbeitung der Daten möglich ist. Hierdurch wird eine deutliche Performance-Optimierung erzielt. Werden im Rahmen einer vorhergehenden Auswertung/Befundung mehrere Bildbereiche als interessierende Bildbereiche klassifiziert, beispielsweise im Rahmen eines Hauptbefunds und eines Nebenbefunds, so werden diese sukzessive entsprechend einer vorgegebenen Gewichtung, beispielsweise durch die Klassifizierung als Hauptbefund und Nebenbefund, geladen.

Zweckdienlicherweise wird die Bildladestrategie weiterhin unter Berücksichtigung des zu untersuchenden Objekts bestimmt, also insbesondere organspezifisch. Hierzu wird wiederum insbesondere auf Daten des Indexes zurückgegriffen, beispielsweise die Kontextdaten. Dadurch besteht beispielsweise die Möglichkeit, zunächst nur die für das jeweilige interessierenden Organ relevante Bilddaten zu laden. Wenn beispielsweise die interessierende anatomische Region eine Knochenstruktur ist, werden über die angepasste Ladestrategie nur derartige Daten geladen, die überhaupt anhand ihrer Struktur, wie beispielsweise Grauwerte, infrage kommen.

Schließlich werden zur Bestimmung der angepassten Ladestrategie auch Untersuchungsziele, die sogenannte "reason for study" ausgewertet. Dieser Ausgestaltung liegt die Überlegung zugrunde, solche Daten zunächst zu laden, die am ehesten Informationen für das Untersuchungsziel enthalten können.

Ergänzend wird weiterhin vorzugsweise durch das Lademodul eine Analyse der Arbeitsabläufe vorgenommen, die für die Bildbearbeitung vorgesehen sind. Dabei wird bei der Abarbeitung eines ersten Arbeitsschrittes bereits der nächste Arbeitsschritt ermittelt und die für diesen Arbeitsschritt relevanten Daten vorgeladen. Die Daten werden daher entsprechend der angepassten Ladestrategie sukzessiv in Abhängigkeit der Abfolge der Arbeitsabläufe geladen.

Zusammenfassend ist daher die optimierte Ladestrategie und damit das Lademodul dahingehend ausgelegt, eine Vielzahl von Informationen aus den Metadaten sowie aus den Kontextdaten als auch aus der Analyse von Arbeitsabläufen/Prozessschritten gezielt auszuwerten, um aus den umfassenden Bilddaten eines jeweiligen Objekts die für die Bildbearbeitung relevanten Bilddaten zu identifizieren und gezielt diese zunächst zu laden, so dass ein möglichst sofortiges Bearbeiten der Bilddaten ermöglicht ist.

Ein wesentlicher Gesichtspunkt hierbei ist die Erstellung des Index, welcher vorzugsweise alle für die Ermittlung der angepassten Ladestrategie relevanten Informationen enthält, nämlich insbesondere die Metadaten sowie die Kontextdaten zu den Bilddaten einer Vielzahl von untersuchten Objekten. Zweckdienlicherweise greift das Lademodul zur Bestimmung der angepassten, performance-optimierten Ladestrategie ausschließlich auf die im Index hinterlegten Informationen zurück.

Ein Ausführungsbeispiel der Erfindung wird im Folgenden anhand der Figuren näher erläutert. Diese zeigen:
- FIG 1: In einer Blockbild-Darstellung ein medizinisches Bilddaten-Verarbeitungssystem, anhand dessen das Verfahren zum Laden von medizinischen Bilddaten näher erläutert wird, sowie
- FIG 2: ein vereinfachtes Schaubild zur Darstellung der Bestimmung einer angepassten Ladestrategie.

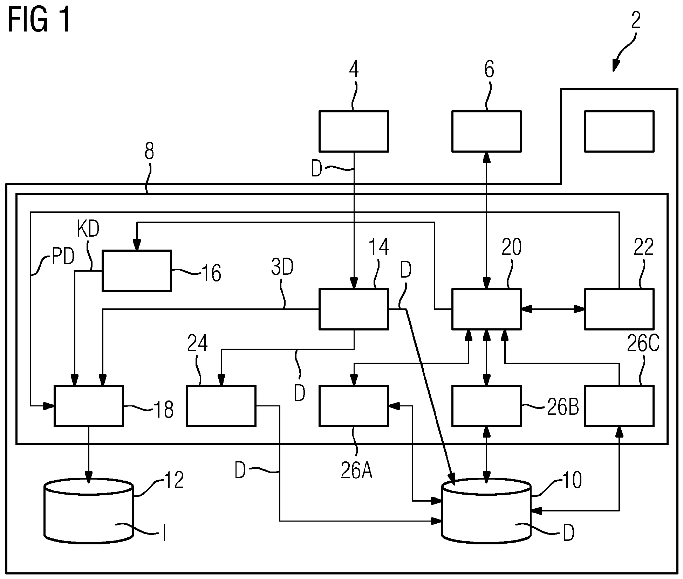

Das in der FIG 1 dargestellte medizinische System ist beispielsweise Teil eines Krankenhaus-Bilddatensystems oder bildet dieses. Dieses umfasst einen Server 2 zur Datenbearbeitung von Bilddaten D, die vorzugsweise aus unterschiedlichen medizinischen bildgebenden Einheiten, den sogenannten Modalitäten 4 stammen. An diesem Server 2 sind vorzugsweise eine Vielzahl von einzelnen Client-Rechnern 6 angeschlossen. In der FIG ist zu Illustrationszwecken lediglich eine Modalität 4 sowie ein Client-Rechner 6 dargestellt.

Auf den Server 2 ist ein Lademodul 8 als ein ausführbares Software-Programm installiert, über das das hier beschriebene Laden von Bilddaten D gesteuert wird. Der Server 2 umfasst weiterhin einen Bilddatenspeicher 10, in dem Bilddaten D von verschiedenen mittels der Modalitäten 4 untersuchten Objekten abgespeichert sind. Schließlich enthält der Server 2 auch einen Indexspeicher 12, in dem ein Index I gespeichert ist. Sowohl der Bilddatenspeicher 10 als auch der Indexspeicher 12 können hardwaretechnisch getrennt von dem Server 2 mit diesem über eine Datenleitung verbunden sein.

Das Lademodul 8 ist wiederum in mehrere funktionale Module unterteilt. Dies sind
- ein Datentyp-Zuordnungsmodul 14, mit dessen Hilfe zu den Bilddaten D eines jeweiligen Objekts ein Datentyp zugeordnet wird,
- ein Analysemodul 16,
- ein Indexersteller 18,
- ein Ablaufmodul 20,
- ein Prozessschritt-Analysemodul 22,
- ein Datenaufbereitungsmodul 24 sowie
- mehrere Ladeeinheiten 26A bis 26C.

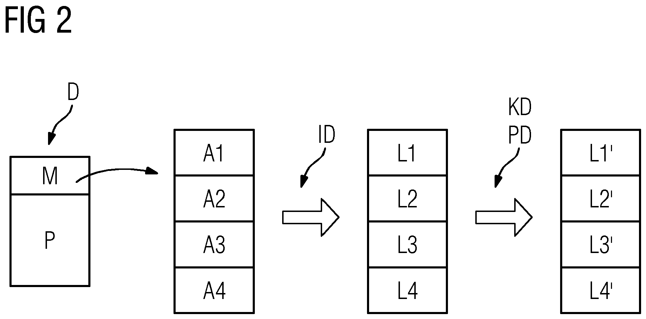

Von der Modalität 4 werden die Bilddaten D dem Lademodul 8 übermittelt. Die Bilddaten D weisen generell Pixeldaten P sowie Metadaten M auf, die den sogenannten "Header" darstellen. Das Datentyp-Zuordnungsmodul 14 analysiert die Metadaten M und ordnet anhand der darin enthaltenen Informationen die Bilddaten D einem speziellen Daten-Typ A1....A4 zu (vgl. FIG 2). Zusammen mit weiteren aus den Bilddaten D extrahierten Informationen übermittelt das Zuordnungsmodul 14 Indexdaten ID an den Indexersteller 18.

Wie weiterhin aus FIG 2 zu entnehmen ist, ist jedem Datentyp A1....A4 jeweils eine Default-Ladestrategie L1....L4 zugeordnet.

Die von der Modalität 4 erhaltenen Bilddaten werden vom Zuordnungsmodul 14 zum Abspeichern im Bilddatenspeicher 10 an diesen weitergeleitet.

Für den Fall, dass die Bilddaten D noch nicht in einem gewünschten, standardisierten Format vorliegen, ist eine Weiterleitung der Bilddaten D an das Datenaufbereitungsmodul 24 vorgesehen, in dem die Bilddaten D in ein gewünschtes Format gebracht werden. Insbesondere betrifft dies die Umwandlung in das WSI-Format mit der kachelpyramidenförmigen Datenstruktur gemäß dem DICOM-Standard, sofern die Daten noch nicht in diesem Format vorliegen und falls die Bilddaten D diesem Daten-typ A3 zugeordnet wurden.

Anhand der Index-Daten ID wird mit Hilfe des Indexerstellers 18 ein Index I erstellt bzw. ein bereits bestehender Index weiter geführt. Bei diesem Index I handelt es sich im Wesentlichen um eine Zuordnungstabelle, aus der zu einem jeweiligen zu einem Objekt gehörigen Bilddatensatz eine Indizierung und damit Zuordnung von verschiedenen Informationen vorgenommen wird. In dieser Indextabelle sind insbesondere alle für die Ermittlung einer angepassten Ladestrategie L1'....L4' sinnvollen oder erforderlichen Informationen sowie Informationen über den Speicherort der Bilddaten etc. hinterlegt.

Der Indexersteller 18 erhält weiterhin Kontextdaten KD vom Analysemodul 16. Das Analysemodul 16 ist ein Postprocessing-Analysemodul, welches die Ergebnisse einer vorhergehenden Auswertung der Bilddaten D auswertet und aus dieser relevante Daten extrahiert. So ist beispielsweise im Rahmen einer ersten Analyse (Befundung) in den Bilddaten D bereits entweder automatisch oder durch das medizinische Personal ein interessierender Bildbereich (ROI, Region Of Interest) identifiziert worden. Das Analysemodul 16 wertet diesen Erstbefund aus und ermittelt hieraus Kontextdaten KD, beispielsweise die Identifizierung der Datenbereiche, die den ROI zeigen. Diese Kontextdaten KD werden in der Indextabelle hinterlegt, sobald sie vorliegen. Im Laufe von Folgeuntersuchungen wird der Index vorzugsweise kontinuierlich ergänzt und gepflegt. Auch werden insbesondere Informationen über einen Hauptbefund und Nebenbefund über das Analysemodul 18 extrahiert und dem Indexersteller 18 übermittelt. Mit Hilfe dieser Kontextdaten KD wird die Default-Ladestrategie L1... L4 verbessert und eine angepasste Ladestrategie L1'....L4' bestimmt, wie in FIG 2 vereinfacht dargestellt ist.

Eine Bildbearbeitung oder auch Bildauswertung der Bilddaten D mit Hilfe eines Bildbearbeitungsprogramms (Applikation) erfolgt üblicherweise auf Anforderung von einem Client-Rechner 6 auf dem Server 2. Vom Bildbearbeitungsprogramm (Applikation) ergeht eine entsprechende Anfrage an das Ablaufmodul 20, um die benötigten Bilddaten D insbesondere in einen Arbeitsspeicher zu laden. Das Ablaufmodul 20 greift auf den Indexspeicher 12 zu (in der FIG nicht näher dargestellt) und ermittelt aus den im Index I enthaltenen Informationen eine angepasste Ladestrategie L1'....L4' zum Laden der gewünschten Bilddaten D aus dem Bilddatenspeicher 10.

Vorzugsweise wird hierbei in einem ersten Schritt anhand des Datentyps eine der Default-Ladestrategien L1....L4 ausgewählt, wobei jeder Default-Ladestrategie L1 ....L4 eine der Ladeneinheiten 26A bis 26C zugewiesen ist (in FIG 1 sind beispielhaft nur drei Ladeeinheiten 26A bis 26C dargestellt). Anhand der weiteren im Index I enthaltenen Informationen, insbesondere den Kontextdaten KD wird diese vordefinierte Default-Ladestrategie verfeinert und verbessert. Anschließend werden die Bilddaten D entsprechend dieser verfeinerten, angepassten Ladestrategie L1'.... L4' über die entsprechend dem Datentyp A1....A4 ausgewählte Ladeeinheit 26A bis 26C geladen und an den Client-Rechner 6 übermittelt.

Die Ergebnisse der mit dem Bildbearbeitungsprogramm entweder automatisch oder auch manuell durchgeführten Analyse der Bilddaten D werden im Ausführungsbeispiel über das Ablaufmodul 20 dem Analysemodul 16 zur Verfügung gestellt, welches die vorgenommene Auswertung der Bilddaten D sogleich analysiert und relevante Daten als Kontextdaten KD extrahiert und dem Indexersteller 18 übermittelt.

Die Auswerteprogramme arbeiten üblicherweise eine definierte Abfolge von Arbeitsschritten ab. Diese definierte Abfolge wird beispielsweise von einem separaten, hier nicht näher dargestellten Auswerte-Manager definiert. Über das Prozessschritt-Analysemodul 22 wird die Abfolge dieser einzelnen Arbeitsschritte analysiert und ausgewertet. Das Ergebnis dieser Auswertung wird wiederum dem Ablaufmodul 22 übermittelt, so dass insbesondere während der Abarbeitung eines Arbeitsschrittes N im Hintergrund bereits die Daten für den nachfolgenden Arbeitsschritt N+1 geladen werden.

Oftmals wird eine vorgegebene Abfolge von Arbeitsschritten nicht vollständig innerhalb einer Arbeitssitzung abgearbeitet. Um dies zu berücksichtigen übermittelt das Prozess-schritt-Analysemodul 22 an den Indexersteller 18 Prozessdaten PD, die Informationen über die auf die Bilddaten D angewandte Applikation sowie den zuletzt durchgeführten Applikationsschritt enthalten. Diese werden ebenfalls in den Index als weitere Kontextdaten KD eingetragen und bei der nächsten Ladeanforderung von dem Ablaufmodul 20 für die Bestimmung eines angepassten Ladealgorithmus L1'...L4'berücksichtigt.

Das hier beschriebene verbesserte Ladeverfahren von Bilddaten D zeichnet sich dadurch aus, dass die Leistung des Servers 2 und damit auch aller darauf ablaufender Applikationen/Bildbearbeitungsprogrammen verbessert ist. Dies erfolgt zum einen durch die Anwendung einer speziellen auf den jeweiligen Datentyp zugeschnittenen Default-Ladestrategie L1....L4, die durch einen speziellen Ladealgorithmus repräsentiert ist. Ein wesentlicher Gesichtspunkt ist weiterhin darin zu sehen, dass diese Default-Ladestrategie durch Kontextdaten KD spezifiziert und eine angepasste Ladestrategie L1'....L4' ermittelt wird. Wesentlich hierbei ist die Identifizierung eines interessierenden Bildbereichs (ROI), so dass dem Arzt bei einem Postprocessing als Erstes immer ein relevanter interessierender Bereich angezeigt wird. Als Beispiel kann eine quantitative Auswertung der Lungendichte herangezogen werden. Diese basiert auf einer sogenannten Thorax CT-Studie. Während ohne spezielle Ladestrategie die Studie heute dem Arzt in einem Standardlayout unsegmentiert präsentiert wird, wird dem Arzt mit dem hier beschriebenen Verfahren mit der optimierten Ladestrategie als erstes eine 3D-Darstellung der segmentierten Lunge präsentiert. Dies wird dadurch erreicht, dass der interessierende Bereich (ROI Lunge) im gemeinsamen Index I eingetragen und als Erstes geladen wird.

## Patentansprüche

1. Verfahren zum automatisierten Laden von medizinischen Bilddaten (D) aus einem zentralen Bilddaten-Speicher in einen Arbeitsspeicher mit Hilfe eines Lademoduls (8), um sie einem Bildbearbeitungsprogramm zur Verfügung zu stellen, wobei die Bilddaten (D) aus Rohdaten eines medizinischen bildgebenden System bei der Aufnahme eines Objekts erzeugt sind und die einem jeweiligen Objekt zugeordneten Bilddaten (D) jeweils Metadaten (M) sowie Pixel-Daten (P) umfassen,
**dadurch gekennzeichnet,**
- **dass** die Bilddaten (D) mit Hilfe eines Datentyp-Zuordnungsmoduls (14) aus mehreren möglichen Datentypen (A1....A4) einem Datentyp (A1....A4) zugeordnet werden, wobei der Datentyp (A1....A4) bestimmt wird aus
a) Volumen-Bilddaten basierend auf einzelnen Schichtaufnahmen als erster Datentyp (A1),
b) Volumen Bilddaten basierend auf Multiframe-Bilddaten als zweiter Datentyp (A2),
c) Bilddaten entsprechend dem Whole Slide Imaging-Standard (WSI) innerhalb des DICOM-Standards als dritter Datentyp (A3),
d) Projektions-Bilddaten als vierter Datentyp (A4),
- **dass** anschließend aus mehreren datentypspezifischen Default-Ladestrategien (L1....L4) eine dem ermittelten Datentyp (A1....A4) zugewiesene Default-Ladestrategie ausgewählt wird,
- **dass** den Bilddaten (D) zusätzlich Kontextdaten (KD) zugewiesen sind und anhand der Kontextdaten die Default-Ladestrategie spezifiziert und eine angepasste Ladestrategie (L1'**...**.L4') bestimmt und für das Laden der Bilddaten herangezogen wird, wobei
- zu den Bilddaten (D) von mehreren medizinischen Objekten mit Hilfe eines Indexerstellers (18) ein gemeinsamer Index (I) erstellt wird bzw. ein bestehender Index (I) weitergeführt wird, wobei über den Index (I) den Bilddaten (D) eines jeweiligen Objekts zugehörige Kontextdaten (KD) zugeordnet werden und wobei auf den Index (I) zur Bestimmung der Kontextdaten (KD) zugegriffen wird, und
- **dass** mittels der angepassten Ladestrategie (L1'....L4') die Bilddaten (D) für die Bildbearbeitung in den Arbeitsspeicher geladen werden.

2. Verfahren nach Anspruch 1,
**dadurch gekennzeichnet, dass** der Datentyp (A1....A4) anhand der Metadaten (M) bestimmt wird.

3. Verfahren nach Anspruch 1 oder 2,
**dadurch gekennzeichnet, dass** die Zuordnung des Datentyps (A1....A4) sowie ein Eintrag in den Index (I) vorgenommen wird, sobald die Bilddaten (D) von einem bildgebenden medizinischen Gerät erhalten werden.

4. Verfahren nach einem der Ansprüche 1 bis 3,
**dadurch gekennzeichnet, dass** Informationen aus einer vorhergehenden Auswertung der Bilddaten (D) in den Kontextdaten (KD) hinterlegt werden

5. Verfahren nach einem der Ansprüche 1 bis 4,
**dadurch gekennzeichnet, dass** die angepasste Ladestrategie (L1'....L4') unter Berücksichtigung von Informationen über interessierende Bildbereiche bestimmt wird, die bei einer vorhergehenden Befundung identifiziert wurden, derart, dass die interessierenden Bildbereiche vorgeladen werden.

6. Verfahren nach Anspruch 5,
**dadurch gekennzeichnet, dass** bei mehreren identifizierten Bildbereichen diese entsprechend einer Gewichtung sukzessive geladen werden

7. Verfahren nach einem der Ansprüche 1 bis 6,
**dadurch gekennzeichnet, dass** die angepasste Ladestrategie (L1'....L4') unter Berücksichtigung der Art des zu untersuchenden Objekts organspezifisch bestimmt wird, derart, dass nur die für das zu bearbeitende Objekt relevanten Daten geladen werden, die anhand ihrer Struktur, wie beispielweise Grauwerte, infrage kommen.

8. Verfahren nach einem der Ansprüche 1 bis 7,
**dadurch gekennzeichnet, dass** zur Bestimmung der ange-passten Ladestrategie (L1'....L4') Untersuchungsziele aus-gewertet werden.

9. Verfahren nach einem der vorhergehenden Ansprüche,
**dadurch gekennzeichnet, dass** für eine Bildbearbeitung auf dem Bildbearbeitungsprogramm vordefinierte Arbeitsabläufe hinterlegt sind und die angepasste Ladestrategie (L1'....L4') durch die Abfolge der Arbeitsabläufe bestimmt wird.

10. Vorrichtung mit einer Rechnereinheit (2) mit einem darauf installierten Lademodul (8) zur Durchführung des Verfahrens nach einem der vorhergehenden Ansprüche.

11. Vorrichtung nach Anspruch 10, bei der das Lademodul (8) aufweist
- ein Datentyp-Zuordnungsmodul (14) zur Zuordnung eines Datentyps zu den Bilddaten (D),
- einen Indexersteller (18) zur Erstellung eines Index (I),
- ein Analysemodul (16) zur automatischen Extraktion von Kontextdaten (KD) aus einer vorhergehenden Auswertung der Bilddaten (D),
- ein Ablaufmodul (20) zur Steuerung des Ladevorgangs der Bilddaten (D), das anhand der im Index (I) hinterlegten Informationen eine angepasste Ladestrategie (L1'....L4') zum Laden der Bilddaten (D) aus einem Bilddatenspeicher (10) bestimmt.

## Claims

1. Method for automatically loading medical image data (D) from a central image data memory into a working memory with the aid of a load module (8) in order to make said data available to an image processing program, wherein the image data (D) are generated from raw data of a medical imaging system during the scanning of an object and the image data (D) assigned to a respective object in each case comprises metadata (M) and pixel data (P),
**characterised in that**
- the image data (D) is assigned with the aid of a data type assignment module (14) to one data type (A1...A4) out of a plurality of possible data types (A1...A4), wherein the data type (A1...A4) is determined from
a) volume image data based on individual slice images as first data type (A1),
b) volume image data based on multiframe image data as second data type (A2),
c) image data conforming to the whole slide imaging (WSI) standard within the DICOM standard as the data type (A3),
d) projection image data as fourth data type (A4),
- subsequently a default loading strategy assigned to the identified data type (A1...A4) is selected out of a plurality of data-type-specific default loading strategies (L1...L4),
- the image data (D) is additionally assigned context data (KD) and the default loading strategy is specified and a modified loading strategy (L1'...L4') is determined on the basis of the context data and utilised for loading the image data, wherein
- a shared index (I) is created for the image data (D) of a plurality of medical objects with the aid of an index generator (18) or an existing index (I) is incremented, wherein associated context data (KD) is assigned to the image data (D) of a respective object via the index (I) and wherein the index (I) is accessed in order to determine the context data (KD), and
- the image data (D) is loaded into the working memory for the image processing by means of the modified loading strategy (L1'...L4').

2. Method according to claim 1,
**characterised in that** the data type (A1...A4) is determined on the basis of the metadata (M).

3. Method according to claim 1 or 2,
**characterised in that** the data type (A1...A4) is assigned and an entry made in the index (I) as soon as the image data (D) is received from a medical imaging device.

4. Method according to one of claims 1 to 3,
**characterised in that** information from a preceding evaluation of the image data (D) is stored in the context data (KD).

5. Method according to one of claims 1 to 4,
**characterised in that** the modified loading strategy (L1'...L4') is determined taking into account information relating to imaging regions of interest which were identified in a preceding assessment such that the imaging regions of interest are preloaded.

6. Method according to claim 5,
**characterised in that** where a plurality of imaging regions are identified these are loaded in succession according to a weighting.

7. Method according to one of claims 1 to 6,
**characterised in that** the modified loading strategy (L1'...L4') is determined in an organ specific manner taking into account the type of object that is to be examined such that only the data that is relevant to the object that is to be processed is loaded, which data comes into consideration on the basis of its structure, such as greyscale values for example.

8. Method according to one of claims 1 to 7,
**characterised in that** the examination objectives are evaluated in order to determine the modified loading strategy (L1'...L4').

9. Method according to one of the preceding claims,
**characterised in that** workflows predefined for image processing on the image processing program are stored and the modified loading strategy (L1'...L4') is determined by the sequence of the workflows.

10. Device comprising a computer unit (2) having a load module (8) installed thereon for performing the method according to one of the preceding claims.

11. Device according to claim 10, wherein the load module (8) comprises
- a data type assignment module (14) for assigning a data type to the image data (D),
- an index generator (18) for creating an index (I),
- an analysis module (16) for automatically extracting context data (KD) from a preceding evaluation of the image data (D),
- a workflow module (20) for controlling the loading of the image data (D) and determining on the basis of the information stored in the index (I) a modified loading strategy (L1'...L4') for loading the image data (D) from an image data memory (10).

## Revendications

1. Procédé de chargement automatisé de données (D) d'images médicales d'une mémoire centrale de données d'images à une mémoire de travail, à l'aide d'un module (8) de chargement, pour les mettre à disposition d'un programme de traitement d'images, les données (D) d'images étant produites à partir de données brutes d'un système médical donnant des images à l'enregistrement d'un objet et comprenant des données (D) d'images, respectivement des métadonnées (M) ainsi que des données (P) de pixels associées à un objet respectif,
**caractérisé**
- **en ce que** l'on associe, à l'aide d'un module (14) d'association de type de données, les données (D) d'images à un type (A1... .A4) de données, parmi plusieurs types (A1... .A4) de données possibles, en déterminant le type (A1... .A4) de données à partir
a) de données d'images en volume reposant sur diverses radiographies panoramiques comme premier type (A1) de données,
b) de données d'images en volume reposant sur des données d'images multitrames comme deuxième type (A2) de données,
c) de données d'images correspondant à la Whole Slide Imaging Standard (WSI) dans le DICOM-Standards comme troisième type (A3) de données,
d) de données d'images de projection comme quatrième type (A4) de données,
- **en ce qu'**ensuite, on choisit parmi plusieurs stratégies (L1... .L4) de chargement de défaut spécifiques au type de données, une stratégie de chargement de défaut affectée au type (A1... .A4) de données déterminé,
- **en ce que** l'on affecte aux données (D) d'images, en plus des données (KD) de contexte et on précise, à l'aide des données de contexte, la stratégie de chargement de défaut et on détermine une stratégie (L1'... .L4') de chargement adaptée et on en tire partie pour le chargement des données d'images, dans lequel
- on établit, pour les données (D) d'images de plusieurs objets médicaux, à l'aide d'un indexeur (18), un index (I) commun, ou on continue avec un index (I) existant, dans lequel on associe, par l'intermédiaire de l'index (I), aux données (D) d'images d'un objet respectif, des données (KD) de contexte qui lui appartiennent et dans lequel on accède à l'index (I) pour déterminer les données (KD) de contexte et
- **en ce que**, au moyen de la stratégie (L1'... .L4') de chargement adaptée, on charge les données (D) d'images pour le traitement d'images dans la mémoire de travail.

2. Procédé suivant la revendication 1,
**caractérisé en ce que** l'on détermine le type (A1... .A4) de données à l'aide des métadonnées (M).

3. Procédé suivant la revendication 1 ou 2,
**caractérisé en ce que** l'on effectue l'association du type (A1... .A4) de données ainsi que l'entrée dans l'index (I) dès que les données (D) d'images sont obtenues d'un appareil médical donnant une image.

4. Procédé suivant l'une des revendications 1 à 3,
**caractérisé en ce que** l'on mémorise, dans les données (KD) de contexte, des informations d'une exploitation précédente des données (D) d'images.

5. Procédé suivant l'une des revendications 1 à 4,
**caractérisé en ce que** l'on détermine la stratégie (L1'... .L4') de chargement adaptée en tenant compte d'informations sur des parties d'images intéressantes, qui ont été identifiées dans une recherche précédente, de manière à charger à l'avance les parties d'images intéressantes.

6. Procédé suivant la revendication 5,
**caractérisé en ce que**, lorsqu'il y a plusieurs parties d'images identifiées, on les charge successivement, conformément à une pondération.

7. Procédé suivant l'une des revendications 1 à 6,
**caractérisé en ce que** l'on détermine la stratégie (L1'... .L4') de chargement adaptée d'une manière spécifique à un organe, en tenant compte du type de l'objet à étudier, de manière à ne charger que les données pertinentes pour l'objet à traiter, qui sont à prendre en considération au moyen de leur structure, comme par exemple de leur valeur de gris.

8. Procédé suivant l'une des revendications 1 à 7,
**caractérisé en ce que**, pour la détermination de la stratégie (L1'... .L4') de chargement adaptée, on exploite des cibles de recherche.

9. Procédé suivant l'une des revendications précédentes, **caractérisé en ce que**, pour un traitement d'images sur un programme de traitement d'images, on mémorise des processus de travail définis à l'avance et on définit la stratégie (L1'... .L4') de chargement adaptée par la succession des processus de travail.

10. Installation comprenant une unité (2) informatique, ayant un module (8) de chargement qui y est installé, pour effectuer le procédé suivant l'une des revendications précédentes.

11. Installation suivant la revendication 10, dans laquelle le module (8) de chargement a
- un module (14) d'association de type de données pour associer un type de données aux données (D) d'images,
- un indexeur (18) pour établir un index (I),
- un module (16) d'analyse pour extraite automatiquement des données (KD) de contexte d'une exploitation précédente des données (D) d'images,
- un module (20) de déroulement pour commander l'opération de chargement des données (D) d'images, qui détermine, à l'aide des informations mémorisées dans l'index (I), une stratégie (L1'... .L4') de chargement adaptée au chargement des données (D) d'images, à partir d'une mémoire (10) de données d'images.
